# EUROPEAN PATENT SPECIFICATION

(11) **EP 4 517 357 B1**
(45) Date of publication and mention of the grant of the patent: **18.02.2026**
(21) Application number: 23275131.3
(22) Date of filing: 31.08.2023
(51) Int. Cl.: G01R 33/38, G01R 33/3815

(54) **SUPPORT ELEMENT AND MAGNET SUPPORT STRUCTURE**
TRÄGERELEMENT UND MAGNETTRÄGERSTRUKTUR
ÉLÉMENT DE SUPPORT ET STRUCTURE DE SUPPORT D'AIMANT

(43) Date of publication of application: 05.03.2025
(73) Proprietor: Siemens Healthcare Limited, Camberley, Surrey GU15 3YL (GB)
(72) Inventor: SIMPKINS, Michael, Finstock, Chipping Norton, OX7 3DW (GB)
(74) Representative: Siemens Healthineers Patent Attorneys

(56) References cited:
- CN-A- 108 447 644
- CN-U- 216 133 730
- GB-A- 2 503 448
- US-A- 5 034 713
- US-A1- 2017 097 397
- SUMAN NAVNEET ET AL: "Composite support system for 1.5 T whole-body MRI cryostat", MATERIALS TODAY: PROCEEDINGS, vol. 62, 2 April 2022 (2022-04-02), NL, pages 309 - 317, XP093127264, ISSN: 2214-7853, DOI: 10.1016/j.matpr.2022.03.374

## Description

Independent of the grammatical term usage, individuals with male, female or other gender identities are included within the term.

Magnets for magnetic resonance devices typically operate at very low temperature levels, for example temperature levels in the range of 3 K to 5 K. In order to maintain the magnet at these low temperature levels as efficiently as possible, the magnets are suspended from a vacuum chamber. The vacuum chamber may also serve as a support structure preventing the magnet from moving when electromagnetic forces are acting on the magnet during a magnetic resonance measurement, but also during transport of the magnetic resonance device.

Recent developments have led to lightweight, serially bonded magnet coil structures. At the same time, increasing material costs and decreasing availability of cryogens used for cooling the magnets have favoured the development of so called "dry" magnetic resonance devices. In a "dry" magnetic resonance device, the magnet is cooled via thermal conduction rather than being immersed in a liquid cryogen contained within a dedicated cryogen vessel. The magnets are typically suspended from the vacuum chamber using suspension elements. However, a tension that needs to be applied to these suspension elements may cause a distortion of lightweight magnets, which is detrimental to a homogeneity of magnetic fields generated by the magnets.

This problem is worsened as a magnetic field strength of the magnet, and thus a mass of the magnet, increases. For example, a low field 0.5 T magnet usually requires less tension than a 1.5 T magnet. High field magnets, such as 3 T magnets or 7 T magnets, are significantly heavier and will require an even higher tension. With increasing tension loads, a stiffness or rigidity of the support structure needs to increase as well, thus increasing the mass and overall complexity.

US 2017/097397 A1 describes a support structure for a magnet that is configured to maintain coil sections of the magnet in fixed axial positions while allowing a radial expansion of the coil sections.

It is an object of the present invention to reduce weight, but also complexity, of magnet support structures for magnetic resonance devices.

This objective is achieved by a support element, a magnet support structure, a magnetic resonance device, and a computer-implemented method for designing a support element according to the invention. Further advantageous embodiments are specified in the dependent claims.

The inventive support element is configured to support a cold mass of a magnetic resonance device. The support element comprises at least one attachment point, a plurality of displaceable sections and a plurality of fixed sections. In a preferred embodiment, the cold mass is mechanically connected or attached to the plurality of fixed sections.

A cold mass of the main magnet can be any structure maintained a temperature level close to a superconducting temperature of a main magnet of the magnetic resonance device. For example, the cold mass may comprise or represent the main magnet and/or a cryogen vessel of the magnetic resonance device. The cold mass may represent an inert mass.

The support element may be configured to reduce or avoid a deforming or warping of an inert mass mechanically connected to the plurality of fixed sections when a predefined force is applied to the at least one attachment point of the support element.

A main magnet of a magnetic resonance device may comprise one or more superconducting magnets. In a preferred embodiment, the main magnet comprises one or more solenoidal or cylindrical superconducting coils. The one or more solenoidal or cylindrical superconducting coils may be rotationally symmetric or comprise rotationally symmetric bodies. According to an embodiment, each solenoidal or cylindrical superconducting coil comprises an axis of rotational symmetry arranged on an axis of rotational symmetry of the main magnet. The one or more solenoidal coils of the main magnet may be serially bonded. For example, the one or more solenoidal coils of the main magnet may be mechanically connected via materially bonds and/or spacers. The main magnet may comprise a dedicated support structure configured to carry the main magnet and/or provide support to the main magnet. The term "main magnet" as used herein may comprise one or more solenoidal superconducting coil as well as a dedicated support structure.

The at least one attachment point may represent a structural feature protruding from a surface, particularly a lateral surface, of the support element. Preferably, the at least one attachment point provides a mounting element, e. g. an anchor, an eyelet, a ring, a plate, a rod, a protrusion, or the like, configured for mechanically connecting the support element to a support structure, particularly an outer vacuum chamber. The at least one attachment point may be configured to bear loads or forces associated with a transport and/or an operation of a magnetic resonance device. Particularly, the at least one attachment point may be configured to carry a weight of the support element and the cold mass and/or to transfer forces associated with the transport and/or operation of the magnetic resonance device from the support element to the support structure. The support element may be configured to absorb a share of the predefined force associated with the transport and/or operation of the magnetic resonance device.

According to an embodiment, the support element comprises a plurality of attachment points. The plurality of attachment points may be distributed over a surface, particularly a lateral surface, of the support element.

In a preferred embodiment, the at least one attachment point, the plurality of displaceable sections and the plurality of fixed sections are mechanically connected. According to the invention, the support element comprises the shape of a ring, a hollow cylinder, a hollow prism, a tube, or a disc. Preferably, the plurality of displaceable sections and the plurality of fixed sections are arranged along a tubular shell or wall of the support element or form parts of the tubular shell or wall of the support element. Particularly, displaceable sections of the plurality of displaceable sections and fixed sections of the plurality of fixed sections may be arranged along the tubular shell of the support element in an alternating fashion.

In a preferred embodiment, a displaceable section and/or a fixed section represent a section, a part, or a segment of a ring, a hollow cylinder, a hollow prism, a tube, or a disc. In a preferred embodiment, the plurality of displaceable sections and the plurality of fixed sections form a connected and/or continuous structure. The plurality of displaceable sections may be directly mechanically connected to the plurality of fixed sections. For example, a displaceable section of the plurality of displaceable sections may be screwed, bolted, clamped, welded, and/or glued to a fixed section of the plurality of fixed sections. It is also conceivable that the plurality of displaceable sections and the plurality of fixed sections form a monolithic structure.

A support element shaped like a tube or prism may circumferentially encompass the cold mass of a magnetic resonance device at least partially along an axial direction defined by the cold mass. Thus, the support element may favourably support the cold mass at dedicated points distributed over a circumference of the cold mass, which may ensure an even distribution of forces associated with a transport and/or operation of the magnetic resonance device. A ring-shaped or tubeshaped support element may benefit from an increased stiffness and/or rigidity in comparison to other geometric shapes.

According to the invention, each displaceable section of the plurality of displaceable sections is configured to be reversibly displaced relative to fixed sections of the plurality of fixed sections when a predefined force is applied to the at least one attachment point and the fixed sections of the plurality of fixed sections are configured to remain in a predefined spatial arrangement to each other when the predefined force is applied to the at least one attachment point.

The fixed sections may be configured to maintain a relative spatial position to each other when the predefined force is applied to the at least one attachment point.

According to an embodiment, the fixed sections are configured to maintain a relative spatial position with respect to an inert mass mechanically connected to the plurality of fixed sections when the predefined force is applied to the at least one attachment point. Preferably, the support element is configured to prevent the fixed elements from shifting against each other when the predefined force is applied to the at least one attachment point. Thus, a deformation of an inert mass mechanically connected the plurality of fixed sections may be reduced or avoided. The inert mass may represent a cold mass of a magnetic resonance device.

The predefined force is oriented in at least one spatial direction. However, the predefined force may also comprise a plurality of force components oriented in different spatial directions. The predefined force may be a force associated with an acceleration and/or a deceleration of a magnetic resonance device during a transport. Particularly, the predefined force may represent a suspension load acting on the at least one attachment point. However, the predefined force may also be associated with a shock or impact on a housing or an outer shell of a magnetic resonance device comprising the support element. In one example, the predefined force may correspond to a gravitational force or a multiple of the gravitational force. However, the predefined force may also represent an acceleration force, a retarding force and/or a centrifugal force acting on the at least one attachment point.

A magnitude of the predefined force may be limited to a maximum force expected to act on the attachment point during a transport of the magnetic resonance device comprising the support element. Preferably, the predefined force is smaller than a force required to exceed a fatigue limit or a breaking point of a material of the support element. The material of the support element may be configured to bear the predefined force over an elongated period of time, such as days, weeks, months or years.

The plurality of displaceable sections may be configured to move, bend and/or deform when the predefined force is applied to the at least one attachment point. According to an embodiment, the displaceable sections are displaced by up to 1 mm, up to 2 mm, up to 3 mm, or up to 4 mm when the predefined force is applied to the at least one attachment point. It is also conceivable that the displaceable sections are displaced by at least 1 mm, at least 2 mm, or at least 3 mm when the predefined force is applied to the at least one attachment point.

The fixed sections may be configured to be displaced or moved relative to each other by a minimal distance when the predefined force is applied to the at least one attachment point. Preferably, a displacement of fixed sections is negligible in comparison to a displacement of the displaceable sections when the predefined force is applied to the at least one attachment point. For example, the fixed sections may be considered to remain in a predefined relative spatial arrangement to each other if a spacing or distance of two fixed sections of the plurality of fixed sections is changed by less than 0.5 mm, less than 0.2 mm, or less than 0.1 mm when the predefined force is applied to the at least one attachment point.

The fixed sections may also be considered to remain in a predefined spatial arrangement to each other if each fixed section of the plurality of fixed sections is displaced by a minimal distance when the predefined force is applied to the at least one attachment point. A minimal distance may be significantly smaller than a displacement of a displaceable section when the predefined force is applied to the at least one attachment point. For example, the minimal distance may be less than 0.5 mm, less than 0.2 mm, or preferably less than 0.1 mm. However, the minimal distance may also be less than 15 %, less than 10 %, or less than 5 % of a displacement of a displaceable section when the predefined force is applied to the at least one attachment point.

The displaceable sections may also considered as deformable sections. Preferably, a shape and/or spatial configuration of the displaceable sections may differ from a shape and/or spatial configuration of the fixed sections in such a way, that the support element is deformed at sections corresponding to the displaceable sections and a deformation of fixed sections is avoided when the predefined force is applied to the at least one section. For example, a material thickness and/or a 3-dimensional shape of the displaceable sections may differ from a material thickness and/or a 3-dimensional shape of the fixed sections in such a way, that the support element primarily deforms at the displaceable sections when the predefined force is applied to the at least one section. It is also conceivable that a material composition of the displaceable sections differs from a material composition of the fixed sections. For example, the displaceable sections may comprise or consist of a material having a lower Young's modulus than a material of the fixed sections.

Preferably, the support element is designed in such a way to allow for a deformation or deflection of the plurality of displaceable sections remotely from the plurality of fixed sections. Thus, an inert mass mechanically connected to the plurality of fixed sections may substantially remain unaffected by the predefined force applied to the at least one attachment point.

Preferably, the inventive support element is designed via a computer-implement method. The inventive computer-implemented method for designing the support element comprises the steps:
- performing a load simulation of the support element to determine a deformation pattern of the support element when a predefined force is applied to at least one attachment point, wherein an inert mass is mechanically connected to the support element at a plurality of sections arranged at predefined spatial locations, and
- determining a parameter of the support element based on the deformation pattern to provide a support element comprising a plurality of displaceable sections and a plurality of fixed sections arranged at the predefined spatial locations.

The load simulation may comprise a numerical simulation and/or finite elements simulation. The deformation pattern may comprise an information on a displacement of one or more discrete points or volume elements of the support element.

The deformation pattern may depend on the applied predefined force, but also on properties of the support element and/or the inert mass, such as a weight, a shape, and/or a mechanism used to attach the inert mass to the support element. The deformation pattern may be used to modify and/or determine a parameter or a set of parameters of the support element, such as a shape, a material composition, and/or a spatial configuration. Particularly, the parameter or set of parameters may be modified in such a way to provide a support element comprising a plurality of fixed sections arranged at predefined or desired spatial locations. The predefined or desired spatial locations of the plurality of fixed sections may depend on a shape and/or a mechanical configuration of the inert mass. In a preferred embodiment, the inert mass corresponds to a cold mass of a magnetic resonance device.

The computer-implemented method may be used to optimize the support element with respect to a weight and/or a size of the support element, but also with respect to spatial locations of the plurality of fixed sections. For example, the computer-implemented method may comprise a step of iteratively modifying a parameter or a set of parameters of the support element in dependence of one or more determined deformation patterns. In a preferred embodiment, the optimization of the support element is based on a cost-function, a least-squares method, a Newton's method, a Gauss-Newton algorithm, a La-grange multiplier, or the like.

According to an alternative embodiment, a load simulation may be used to identify a plurality of sections of the support element which exhibit a minimum displacement when a predefined force is applied to at least one attachment point and when the inert mass is mechanically connected to at least one section of the support element.

According to an embodiment of the inventive support element, the plurality of fixed sections is configured to prevent or reduce a deformation of an inert mass connected to the plurality of fixed sections when the predefined force is applied to the at least one attachment point.

An inventive support element may favourably allow for suspension loads acting on the at least one attachment point to be deflected to regions of the support element (i. e. the plurality of displaceable sections) which are configured to be elastically deformed. Thus, a deformation of an inert mass, particularly a main magnet and/or a cryogen vessel, carried by the support element may favourably be reduced or avoided.

Known solutions for supporting the cold mass of a magnetic resonance device tend to be rigid, heavy, and somewhat complex, which increases manufacturing costs, but may also complicates transport. In providing a support element configured to prevent a displacement or deflection of fixed sections mechanically connected to the cold mass, material costs as well as manufacturing efforts associated with the magnet support structure may favourably be reduced.

Precise manufacturing techniques may need to be applied only to a few key locations of the support element, e. g. where an inert mass is to be attached to the support element or where the support element is to be attached to a support structure. Thus, large portions of the support element may favourably be processed using relatively simple and low-cost manufacturing processes such as water jet cutting or casting.

According to an embodiment of the inventive support element, the at least one attachment point is arranged at a displaceable section of the plurality of displaceable sections and mechanically connected to the displaceable section.

As described above, the at least one attachment point may be configured to mechanically connect to a support structure, which may apply or transfer suspension loads and/or other forces to the at least one attachment point. In a preferred embodiment, the at least one attachment point is configured to introduce the suspension loads and/or other forces into the support element.

The at least one attachment point may protrude from a surface of a displaceable element of the plurality of displaceable elements. Preferably, the at least one attachment point protrudes outward from a surface of the support element, e. g. along a radial direction of the support element or a direction leading away from the support element.

In providing a support element comprising at least one attachment point attached to a displaceable section, the at least one attachment point is allowed to move or bend together with the displaceable section when a suspension load and/or another force is applied to it. Thus, requirements regarding a material choice and/or a rigidity of the at least one attachment point, but also the support element, may favourably be reduced.

In a preferred embodiment, the inventive support element comprises at least one connecting element configured to mechanically connect to an inert mass. The at least one connecting element may be embodied in accordance with a connecting element described below. An inert mass may correspond to a cold mass of a magnetic resonance device. In a preferred embodiment, the at least one connecting element is configured to carry the inert mass or suspend the inert mass from the support element.

According to the invention, the at least one connecting element is arranged at a fixed section of the plurality of fixed sections and mechanically connected to the fixed section. The at least one connecting element may be mechanically connected to the support element according to an embodiment described below. However, the support element and the at least one connecting element may also form a monolithic structure.

According to an embodiment, the support element comprises an inert mass, particularly a cold mass of a magnetic resonance device, mechanically connected to the at least one connecting element.

The at least one connecting element may favourably allow for mechanically connecting an inert mass to the support element. As the at least one connecting element is arranged at a fixed section, a deformation or torsion of the inert mass may favourably be reduced or avoided, when the predefined force is applied to the at least one attachment point.

According to an embodiment of the inventive support element, the plurality of displaceable sections comprises a first displaceable section and a second displaceable section. The first displaceable section and the second displaceable section are separated by a fixed section of the plurality of fixed sections.

For example, the fixed section may be arranged between the first displaceable section and the second displaceable section. It is also conceivable that the first displaceable section and/or the second displaceable section are each arranged between two fixed sections of the plurality of fixed sections. According to an embodiment, the plurality of fixed sections and the plurality of displaceable sections may be arranged alternatingly along a segment of the support element. In a preferred embodiment, the number of displaceable sections corresponds to the number of fixed sections.

In providing an alternating arrangement of displaceable sections and fixed sections, mechanically connecting the support element to rotationally symmetric objects of the cold mass, such as the main magnet or the cryogen vessel, may favourably be facilitated. For example, the main magnet may be mechanically connected to different points along an inner circumference of a tubular or ring-shaped support element, thus decreasing a tendency of rolling or twisting of the main magnet in relation to the support element.

According to a further embodiment, the inventive support element comprises at least three displaceable sections and at least three fixed sections.

Preferably, the at least three displaceable sections and the at least three fixed sections are alternatingly distributed along a segment of the support element, particularly a wall or shell of a tubular or ring-shaped support element. The at least three displaceable sections and the at least three fixed sections may be arranged in regular or irregular intervals along the shell of the support element.

A dimension of a displaceable section of the plurality of displaceable sections may differ from a dimension of a fixed section of the plurality of fixed sections. Preferably, a dimension of a displaceable section of the plurality of displaceable sections may exceed a dimension of a fixed section of the plurality of fixed sections.

In increasing a number of fixed sections mechanically connected to the cold mass, a tendency of rolling or twisting of the cold mass due to electromagnetic forces occurring during an operation of a magnetic resonance device may favourably be reduced.

According to a further embodiment, the inventive support element comprises at least three attachment points, preferably four attachment points.

It is conceivable, that each displaceable section of the plurality of displaceable sections comprises exactly one attachment point. However, it is also conceivable that multiple attachment points are arranged at one displaceable section of the plurality of displaceable sections.

According to an embodiment, a first attachment point and a second attachment point are arranged at a first displaceable section of the plurality of displaceable sections, whereas a third attachment point and a fourth attachment point are arranged at a second displaceable section of the plurality of displaceable sections. The plurality of displaceable sections of the support element may comprise a third displaceable section which is spaced or separated from the first attachment point and the second attachment point, but also from any other attachment point of the support element.

In providing a plurality of attachment points configured to mechanically connect to a support structure, a tendency of twisting or rolling of the support element relative to the support structure may favourably be reduced or avoided.

According to an embodiment, the inventive support element comprises the shape of a tube, a ring, or a disc and the at least one attachment point protrudes from a lateral surface of the support element.

A lateral surface of the support element may correspond to an outer surface of the support element. For example, the support element may comprise the shape of a hollow cylinder and the lateral surface may correspond to an outer surface of the hollow cylinder.

The at least one attachment point may be configured according to an embodiment described above. The at least one attachment point may be mechanically connected to the lateral surface of the support element via a form-locking, a force-locking, and/or a material connection. For example, the attachment point may be screwed, bolted, clamped, swaged, welded and/or glued to the support element. In a preferred embodiment, the at least one attachment point and the support element, particularly a displaceable section of the support element, form a monolithic structure.

In providing an inventive support element comprising at least one attachment point according to an embodiment described above, a mechanical connection between the support element and a support structure carrying the support element may favourably be facilitated.

In an embodiment, the inventive support element comprises at least two attachment points oriented at opposing sides of the support element.

A side of the support element may correspond to a segment of a ring, a hollow cylinder, or a tube of the support element. An opposing side may represent a side of the support element arranged at a rotational angle of 160° to 200°, particularly 180°, with respect to a reference side of the support element. For example, a second side arranged at 180° with respect to a first side along a circumference of the support element may be regarded as opposing the first side.

In a further example, the support element comprises a first attachment point and a second attachment point. The first attachment point may be arranged on a first side of the support element and the second attachment point may be arranged on a second side of the support element. The first attachment point may be arranged symmetrically to the second attachment point. Particularly, the first attachment point may be arranged at a first side of the support element and the second attachment point may be arranged at a second side of the support element opposing the first side. It is conceivable that the first attachment point and the second attachment are arranged along a perpendicular to the axis of the support element.

In a preferred embodiment, the inventive support element comprises two attachment points arranged at a first side of the support element and two further attachment points arranged at a second side of the support element opposing the first side.

In arranging a plurality of attachment points on two opposing sides of the support element, a distribution of the plurality of fixed sections over a circumference of the support element may be facilitated and/or homogenised. Particularly, a more uniform distribution of the plurality of fixed points over the circumference of the support element may be achieved.

Furthermore, arranging a plurality of attachment points on two opposing sides of the support element may allow for the suspension elements to be longer which may favourably reduce or minimise a transport of heat energy to the main magnet along the suspension elements. As a further advantage, the inventive support element may allow for an angle between the suspension elements and a gravitational force vector to be modified in such a way to reduce or minimise distortion of the suspension elements and the outer vacuum chamber, but also restrain the main magnet from rotational movement.

According to a preferred embodiment, the support element comprises the shape of a tube or a ring and the plurality of displaceable sections is configured to be displaced along a radial direction of the support element when the predefined force is applied to the at least one attachment point. A vector of the predefined force is oriented in parallel to a vector of the gravitational force. The predefined force may correspond to a gravitational force acting on the at least one attachment point. A gravitational force acting on the at least one attachment point may represent a standard load scenario of the magnetic resonance device.

Preferably, the at least one displaceable section is configured to be displaced away from a geometric centre of the support element when the predefined force is applied to the at least one attachment point. However, it is also conceivable that at least one displaceable section is configured to be displaced towards a geometric centre of the support element when the predefined force is applied to the at least one attachment point force.

The plurality of displaceable sections may be bend and/or comprise a variable material thickness to allow for a deformation or displacement along the radial direction of the support element.

In allowing the plurality of displaceable sections to be displaced along a radial direction of the support element, a deformation and/or or motion of the plurality of displaceable sections may favourably be compensated by a gap provided between the plurality of displaceable elements and a cold mass mechanically connected to the support element.

In allowing the plurality of displaceable sections to be displaced along the radial direction of the support element instead of an axial direction of the support element, a twisting or swaying motion of the support element may favourably be avoided. Furthermore, a displacement of the plurality of displaceable sections along a radial direction of the support element may favourably allow for arranging multiple support elements along an axial direction of a main magnet or a cryogen vessel without causing a mechanical interference between adjacent support elements.

According to an embodiment, the plurality of displaceable sections is configured to be displaced along a radial and/or an axial direction of the support element when the predefined force is applied to the at least one attachment point. In providing a plurality of displaceable sections configured to be displaced along a radial and an axial direction of the support element, the force applied to the at least one attachment point may be absorbed or deflected with lesser overall displacement of the plurality of displaceable sections in a specific direction.

The inventive magnet support structure comprises an outer vacuum chamber, a cold mass, and a support element according to an embodiment described above.

The outer vacuum chamber may form a vessel which is substantially impermeable to fluids, such as a liquid or a gaseous cryogen. The vacuum chamber may be configured to maintain a vacuum in an inner volume encompassed by the vacuum chamber. Preferably, the outer vacuum chamber encompasses other components of the magnet support structure, such as a cryogen vessel, a main magnet, the support element, a thermal shield, and the like. According to an embodiment, the outer vacuum chamber comprises an outer shell, an inner shell and annular end walls connecting the outer shell and the inner shell. Particularly, the outer vacuum chamber may form a double-walled hollow cylinder enclosing the main magnet and the support element between the outer shell, the inner shell and the annular end walls. The outer vacuum chamber may comprise an axis of rotational symmetry oriented in parallel to or corresponding to an axis of rotational symmetry defined by the main magnet, particularly a solenoid coil of the main magnet. The inner shell of the outer vacuum chamber may correspond to a patient bore of a magnetic resonance device.

According to the invention, the at least one attachment point is mechanically connected to the outer vacuum chamber.

In a preferred embodiment, the outer vacuum chamber is configured to provide mechanical support to the support element. For example, the support element may be suspended of or carried by the outer vacuum chamber. In a preferred embodiment, the at least one attachment point is mechanically connected to the outer vacuum chamber via at least one suspension element, particularly a plurality of suspension rods. The support structure may comprise a plurality of attachment points, each attachment point being mechanically connected to the outer vacuum chamber via a suspension element. The suspension element preferably consists of a material with high tensile strength. Examples for suitable materials are metals, particularly stainless steel, or composite materials, such as fibre reinforced plastics, and the like. The suspension element may comprise the shape of a bar, a rod, a pole, but also a band or a wire. A first end and a second end of the suspension element may each comprise an anchor or ferrule. The anchor or ferrule may be attached to the outer vacuum chamber and/or the support element via a suitable mechanical connection, such as a form-locking connection, a force-locking connection and/or a material bond. For example, the anchor or ferrule may be screwed, bolted, clamped, wedged and/or glued to the outer vacuum chamber and/or the support element. However, any suitable mechanical connection may be used.

In providing a support element suspended of the outer vacuum chamber via a plurality of suspension elements, a combined weight force of the support element and the cold mass may favourably be compensated so that balanced suspension forces are achieved at dedicated attachment points.

A suspension element according to an embodiment described above may favourably comprise a small or minimised crosssectional-area. Thus, a transfer of heat energy along the suspension element, but also a weight of the suspension elements, may favourably be reduced.

The plurality of fixed sections is mechanically connected to the cold mass.

The plurality of fixed sections may be connected to the cold via any suitable mechanical connection, e. g. a form-locking, a force-locking and/or a materially connection. For example, the plurality of fixed may be screwed, bolted, clamped and/or glued to the cold mass. However, it is also conceivable that at least one connecting element is provided between the plurality of fixed sections and the cold mass. The connecting element may be mechanically connected to a fixed section of the plurality of fixed sections and the cold mass via any suitable mechanical connection.

According to the invention, the cold mass is spaced from the plurality of displaceable sections.

Preferably, the connecting element is configured to space the plurality of fixed sections from the cold mass. An inner diameter of the support structure may exceed a diameter of the cold mass. For example, an inner diameter of a tubular support structure may exceed an outer diameter of the main magnet and/or an outer diameter of the cryogen vessel. Thus, one or more gaps may be provided between sections of the support element and the cold mass, which may allow for the plurality of displaceable sections to move or bend without contacting the support element.

The inventive magnet support structure shares the advantages of an inventive support element according to an embodiment described above.

According to an embodiment of the inventive magnet support structure, the plurality of fixed sections is mechanically connected to the cold mass via a plurality of connecting elements. The plurality of connecting elements is configured to space the support element from the cold mass.

A connecting element may provide a mechanical connection between a fixed section and the cold mass. The connecting element may be a simple spacer, such as a pin, a bolt, a block, a plate, a ring, a segment of a ring, a hollow cylinder, a sector of a hollow cylinder, or the like. However, the connecting element may also represent a suspension rod. It is conceivable that the connecting element is mechanically connected to the support element and/or the cold mass via a form-locking, a force-locking and/or a materially connection.

Preferably, the plurality of connecting elements comprises at least two connecting elements spaced apart along a circumference of the cold mass in such a way, that a gap is provided between the at least two connecting elements, but also between the support element and the cold mass. According to an embodiment, the plurality of connecting elements comprises at least three connecting elements which are spaced apart along a circumference of the cold mass in such a way, that gaps are provided between the at least three connecting elements along the circumference of the cold mass. In a preferred embodiment, a location of a gap between two connecting elements of the plurality of connecting elements coincides with a location of a displaceable section along a circumference of the support element. Particularly, gaps may be provided between the cold mass and each displaceable section of the plurality of displaceable sections.

In providing gaps between the support element and the cold mass, a contact between the plurality of displaceable elements and the cold mass may favourably be avoided when the at least one attachment point is loaded with a predefined force.

The inventive magnetic resonance device is configured for acquiring magnetic resonance data of an object positioned within an imaging region of the magnetic resonance device.

Preferably, the magnetic resonance device is configured to acquire magnetic resonance image data, particularly diagnostic magnetic resonance image data, from the object positioned within the imaging region. The object may be a patient, particularly a human or an animal.

The inventive magnetic resonance device may represent a closed bore scanner. A closed bore scanner may comprise a substantially cylindrical bore circumferentially enclosing the imaging region. The main magnet of the closed bore scanner may comprise one or more solenoid coils circumferentially encompassing the imaging region along an axial direction or an axis of rotational symmetry of the cylindrical bore. The one or more solenoid coils may comprise a wire having negligible electrical resistance at (or below) a superconducting temperature. A direction of a main magnetic field provided via the main magnet may be oriented substantially in parallel to a direction of access of the object to the imaging region and/or the axial direction of the cylindrical bore.

The inventive magnetic resonance device may represent a "dry" system comprising a minimum of cryogen or no cryogen at all. For example, the inventive magnetic resonance system may comprise one or more small cryogen vessels thermally connected to the main magnet via a solid thermal conductor. The small cryogen vessels may contain a volume of less than 10 l, less than 5 l, or less than 1 l of cryogen. In an embodiment of the inventive magnetic resonance device, a cryogen vessel is omitted. Thus, the main magnet may be cooled entirely via thermal conduction.

It is conceivable that the components of the magnetic resonance device, such as the main magnet, the support element, the magnet support structure, the thermal shield, one or more suspension elements, the cryogen vessel, or the like are thermally connected to the at least one cryocooler, preferably via a solid thermal conductor, a convection loop and/or a heat pipe.

In a "dry" system, the term cold mass may refer to the main magnet. For example, the main magnet may be mechanically connected to the plurality of fixed sections of the support element according to an embodiment described above. Preferably, the main magnet is cooled exclusively via thermal conduction. However, a "dry" magnetic resonance device may still comprise one or more cryogen vessels comprising a cryogen thermally connected to the main magnet. According to an embodiment, the inventive magnetic resonance device comprises a cryogen vessel containing a cryogen, wherein the cryogen vessel is thermally connected to the main magnet via a solid thermal conductor.

In an alternative embodiment, the inventive magnetic resonance device is configured as a "wet" system. A "wet" system may comprise a cryogen vessel containing a fluid or a cryogen with a low boiling point like Argon, Nitrogen, Neon, Helium, or the like. In a "wet" system, the main magnet may be disposed within the cryogen vessel and at least partially immersed in a liquid portion of the cryogen. The term cold mass may refer to the cryogen vessel. For example, the cryogen vessel may be mechanically connected to the plurality of fixed sections of the support element according to an embodiment described above. Alternatively, the support element and the main magnet may be mechanically supported within the cryogen vessel. In this case, the term cold mass may refer to the main magnet, which is mechanically connected to the plurality of fixed sections of the support element according to an embodiment described above.

The magnetic resonance device may comprise at least one cryocooler. The at least one cryocooler may be configured to cool components of the magnetic resonance device. For example, the at least one cryocooler may be configured to cool the main magnet, a thermal shield, the cryogen vessel, parts of the magnet support structure, and the like.

The at least one cryocooler may be configured to provide a temperature close to or lower than a superconducting temperature of a superconducting material of the main magnet. For example, the superconducting temperature of the main magnet may be in the range of 3 K to 100 K, preferably in the range of 3 K to 5 K, 30 K to 60 K, or 60 K to 90 K. The at least one cryocooler may be implemented as a pulse tube refrigerator, a Gifford-McMahon refrigerator, a Stirling cryocooler, a Joule-Thomson cryocooler, or the like. In a preferred embodiment, the at least one cryocooler comprises at least two cooling stages having different temperature levels. Thus, the cold mass and the magnet support structure of the magnetic resonance device may be maintained at different temperature levels.

In a preferred embodiment, the at least one cryocooler is thermally and mechanically connected to the main magnet, the thermal shield, and/or a component of the magnet support structure, particularly the support element. The at least one cryocooler may be configured to maintain the main magnet, the thermal shield, and the component of the magnet support structure at different temperature levels.

It is conceivable that components of the magnetic resonance device, such as the main magnet, the cryogen vessel, the thermal shield, the magnet support structure, and the like, are thermally connected to the cryocooler. Preferably, the components of the magnetic resonance device are thermally connected to the cryocooler via a solid thermal conductor and/or a heat pipe.

A thermal shield may be configured to reduce a transport of heat energy to the main magnet. A transport of heat energy may be characterized by a heat transport mechanism such as thermal radiation, heat conduction, but also heat convection. The thermal shield may circumferentially encompass the main magnet. For example, the thermal shield may form a vessel enclosing the main magnet. In a preferred embodiment, the thermal shield comprises an outer wall, an inner wall and annular end walls connecting the outer wall and the inner wall. Particularly, the thermal shield may form a double-walled hollow cylinder enclosing the main magnet, but also the support element, between the outer wall, the inner wall and the annular end walls. A cylinder axis of the thermal shield may be oriented in parallel to or correspond an axis of rotational symmetry defined by the solenoidal coil of the main magnet.

Preferably, the thermal shield comprises or consists of a material with high electrical and/or high thermal conductivity. For example, the thermal shield may consist of copper or aluminium, particularly a copper alloy or an aluminium alloy. The thermal shield may also comprise gold, platinum, silver, or other materials with high thermal conductivity. In one embodiment, the thermal shield is coated or galvanized with gold, platinum, or other metals with high thermal conductivity.

The thermal shield may comprise a continuous or coherent surface. In an alternative embodiment, the thermal shield may comprise or consist of a plurality of spaced shield elements or rings. The thermal shield may further comprise one or more sections of coiled wire.

In a preferred embodiment, the thermal shield is shaped in such a way to conform to a shape of the main magnet. For example, a surface contour of the thermal shield may imitate or conform to a surface contour of the main magnet.

The inventive magnetic resonance device comprises a magnet support structure according to an embodiment described above.

The inventive magnetic resonance device shares the advantages of the inventive magnet support structure and the inventive support element according to an embodiment described above.

According to an embodiment of the inventive magnetic resonance device, a smallest angle between a line defined by an axial extension of the at least one suspension rod and a gravitational force vector is at least 35°.

The gravitational force vector may be oriented in parallel to a surface normal of a ground surface on which the magnetic resonance device is positioned or installed.

A smallest angle may be independent from a direction of measurement and/or from a coordinate system. For example, the smallest angle may quantify a minimum angle between the line defined by an axis and/or axial extension of the at least one suspension rod and the gravitational force vector, regardless of a rotation or orientation of a coordinate system.

According to an embodiment, the smallest angle between the line defined by the axis of the at least one suspension rod and the gravitational force vector is at least 35°. Preferably, the smallest angle between the line defined by the axis of the at least one suspension rod and a gravitational force vector is at least 40°, 45°, 50°, or 55°.

In providing a magnetic resonance device comprising a magnet support structure suspended via a suspension rod oriented at a shallow angle with respect to a gravitational force vector, a rotation of the magnet support structure about a direction defined by the gravitational force vector may favourably be reduced or avoided. Furthermore, a process of mounting or attaching the support element to the outer vacuum chamber may favourably be facilitated.

Further advantages and details of the present invention may be recognized from the embodiments described below as well as the drawings. The figures show:
Fig. 1 a schematic representation of an embodiment of an inventive magnetic resonance device,
Fig. 2 a schematic representation of an embodiment of an inventive magnetic resonance device,
Fig. 3 a schematic representation of an embodiment of an inventive magnetic resonance device,
Fig. 4 a schematic representation of an embodiment of an inventive magnet support structure,
Fig. 5 a schematic representation of an embodiment of an inventive magnet support structure,
Fig. 6 a schematic representation of an embodiment of an inventive support element.

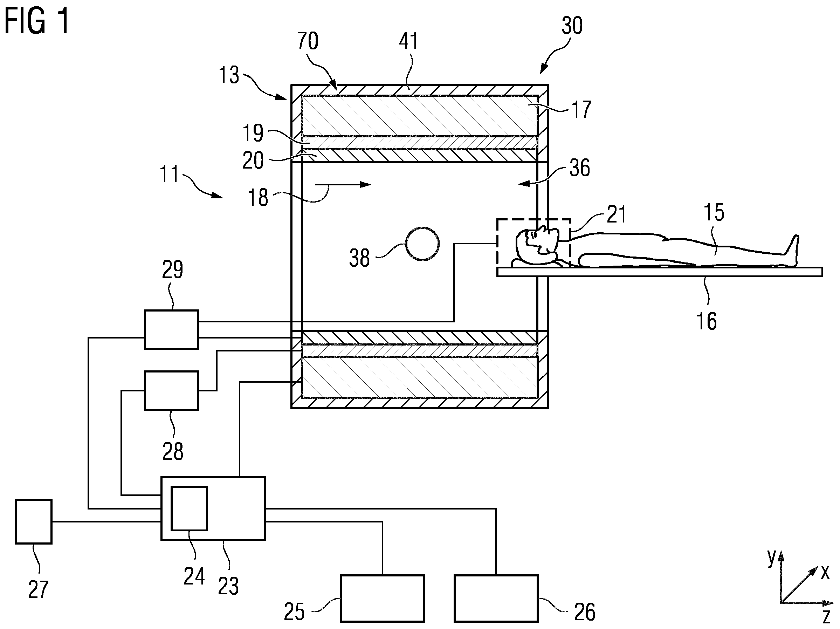

Fig. 1 shows an embodiment of a magnetic resonance device 11 according to the invention. In the illustrated example, the magnetic resonance device 11 comprises a static field magnet 17 or main magnet configured to provide a homogenous, static magnetic field 18 (B0 field) having an imaging volume 38. The static magnetic field 18 permeates an imaging region 36 configured for receiving an imaging object, such as a patient 15. The imaging region 36 may correspond to a patient bore configured for accommodating a patient during a magnetic resonance measurement. The imaging region 36 is encompassed by the main magnet 17 in a circumferential direction.

In the depicted example, the magnetic resonance device 11 comprises a patient support 16 configured to transport the patient 15 into the imaging region 36. Particularly, the patient support 16 is configured to transport a diagnostically relevant body region of the patient 15 into the imaging volume 38 or isocentre of the magnetic resonance device 11. The main magnet 17 and other components of a field generation unit (not shown) of the magnetic resonance device 11 may be concealed in a housing 41.

The magnetic resonance device 11 may comprise a gradient system 19 configured to provide gradient magnetic fields used for spatial encoding of magnetic resonance signals acquired during a magnetic resonance measurement. The gradient system 19 is activated or controlled by a gradient controller 28 via an appropriate current signal. It is conceivable that the gradient system 19 comprises one or more gradient coils configured to generate gradient magnetic fields in different, preferably orthogonally oriented, spatial directions.

The magnetic resonance device 11 may comprise an integrated radiofrequency antenna 20 (i. e. a body coil). The radiofrequency antenna 20 may be operated via a radiofrequency controller 29 that controls the radiofrequency antenna 20 to generate a high frequency magnetic field and emit radiofrequency excitation pulses into the imaging region 36. The magnetic resonance device 11 may further comprise a local coil 21. The local coil 21 may be positioned on or in proximity to the diagnostically relevant region of the patient 15. The local coil 21 may be configured to emit radiofrequency excitation pulses into the patient 15 and/or receive magnetic resonance signals from the patient 15. It is conceivable, that the local coil 21 is controlled via the radiofrequency controller 29.

Preferably, the magnetic resonance device 11 comprises a control unit 23 configured for controlling the magnetic resonance device 11. The control unit 23 may comprise a processing unit 24 configured to process magnetic resonance signals and reconstruct magnetic resonance images. The processing unit 24 may also be configured to process input from a user of the magnetic resonance device 11 and/or provide an output to a user. For this purpose, the processing unit 24 and/or the control unit 23 can be connected to a display unit 25 and an input unit 26 via a suitable signal connection. For a preparation of a magnetic resonance measurement, preparatory information, such as imaging parameters or patient information, can be provided to the user via the display unit 25. The input unit 26 may be configured to receive information and/or imaging parameters from the user.

Of course, the magnetic resonance device 11 may comprise further components and/or functions that magnetic resonance devices usually offer. The general operation of a magnetic resonance device 11 is known to those skilled in the art, so a more detailed description is omitted.

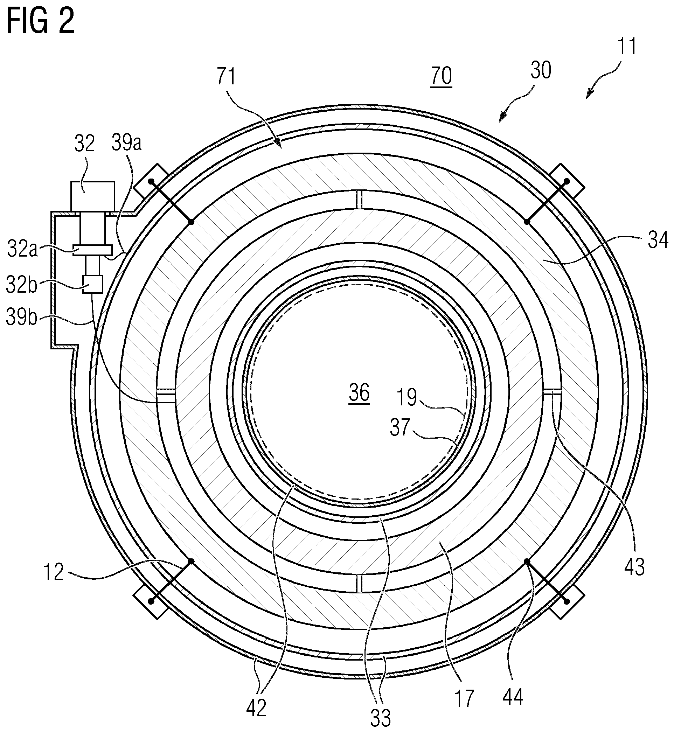

Fig. 2 shows a sectional view of the inventive magnetic resonance device 11 according to Fig. 1. In the depicted example, an outer vacuum chamber 42 provides an enclosure for the components of the magnet support structure 30. The outer vacuum chamber 42 separates a surrounding environment 70 from a vacuum region 71 encompassed by the outer vacuum chamber 42. In a preferred embodiment, the outer vacuum chamber 42 forms a double-walled hollow cylinder comprising an outer shell and an inner shell. The components of the magnet support structure 30 may be housed within or enclosed by the outer shell and the inner shell of the outer vacuum chamber 42. The inner shell of the outer vacuum chamber 42 may form the patient bore 37 encompassing the imaging region 36 in a circumferential direction.

In the illustrated embodiment, the magnet support structure 30 comprises the outer vacuum chamber 42, the support element 34, the four suspension rods 12 and the three connecting elements 43. The support element 34 and the main magnet 17 are enclosed between an outer wall and an inner wall of a thermal shield 33. Furthermore, the main magnet 17 is arranged between the support element 34 and the inner wall of the thermal shield 33 at a transverse section through the magnetic resonance device 11 as depicted in Fig. 2.

Preferably, the outer shell of the vacuum chamber 42 is mechanically connected to the support element 34 via a plurality of suspension rods 12. The suspension rods 12 may extend through passages or holes in the thermal shield 33 to provide a mechanical connection between the outer shell of the outer vacuum chamber 42 and the support element 34.

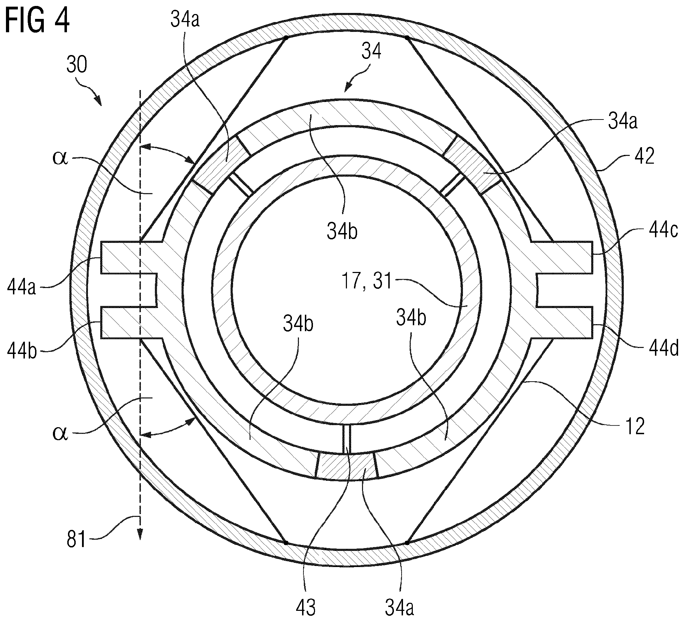

Preferably, the magnetic resonance device 11 comprises a cryocooler 32 mounted on the outer vacuum chamber 42. The cryocooler 32 may be configured to cool the main magnet 17, the thermal shield 33, the support element 34, but also other components of the magnet support structure 30, such as a cryogen vessel 31 (see Fig. 4).

The cryocooler 32 typically comprises a compressor supplying pressurized gas to the cryocooler 32 (not shown). According to the embodiment shown in Fig. 2, the cryocooler 32 includes a cold head comprising a plurality of cooling stages 32a and 32b. Preferably, a first cooling stage 32a of the cold head is thermally connected to the thermal shield 33, whereas a second cooling stage 32b of the cold head is thermally connected to the main magnet 17. According to an embodiment, the first cooling stage 32a provides a temperature level of about 50 K, whereas the second cooling stage 32b provides a temperature level of about 4 K. In "dry" systems, the cooling stages 32a and 32b of the cryocooler 32 can be thermally connected to the thermal shield 33 and the main magnet 17 via solid thermal conductors 39a and 39b. It is also conceivable that the magnetic resonance device 11 comprises one or more small cryogen vessels (not shown) thermally connected to the cryocooler 32 via a solid thermal conductor, a heat pipe, and/or a convective loop. The cryogen vessel may be thermally connected to the main magnet 17 via a heat exchanger and/or a solid thermal conductor 39.

During operation of the magnetic resonance device 11, the thermal shield 33 may be maintained at an intermediate temperature, e. g. a temperature level between 40 K and 60 K, preferably a temperature level of about 50 K. The thermal shield 33 may comprise an electrically conductive material configured for shielding the main magnet 17 from thermal radiation, but also from stray fields of the gradient magnetic fields generated via the gradient system 19.

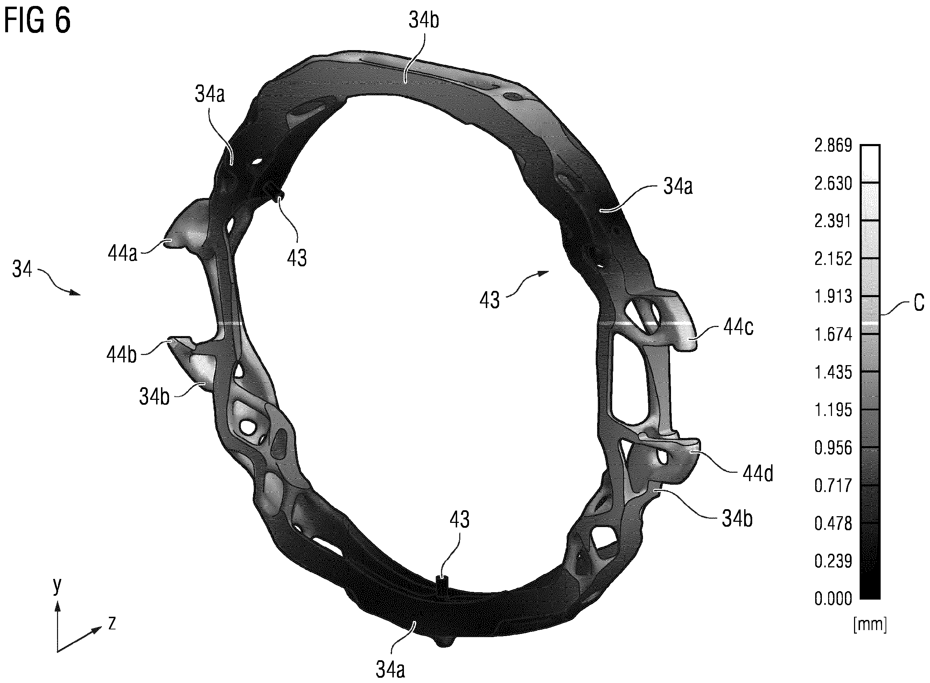

In Fig. 2, the main magnet 17 is mechanically connected to the support element 34 via four connecting elements 43. The connecting elements 43 are spaced from each other along a circumference of the main magnet 17. Thus, gaps are provided between the connecting elements 43. In the depicted example, the gaps coincide with displaceable sections of the support element 34 (see Fig. 6), which can be deformed and/or displaced without contacting the main magnet 17 and/or causing deformations near the fixed sections in proximity to the connecting elements 43. The connecting elements shown in Fig. 2 may be implemented as pins or bolts. Preferably, the connecting elements 43 are configured to carry or hold the main magnet 17 in a predefined position while spacing the main magnet 17 from the support element 34.

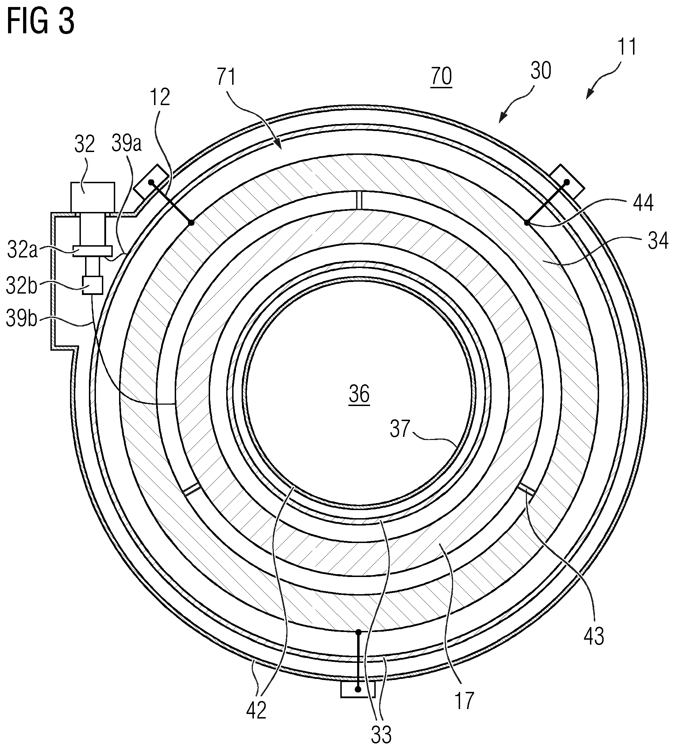

In the embodiment depicted in Fig. 3, the magnet support structure 30 comprises three suspension rods 12 mechanically connecting the outer shell of the outer vacuum chamber 42 to the support element 34. The support element 34 comprises three connecting elements 43 mechanically connected to the main magnet 17. As described in context with Fig. 2, the connecting elements 43 are preferably arranged in proximity to the fixed sections of the support element 34 to avoid a transfer of motion and/or forces from the support element 34 to the main magnet 17 when a force is applied to the attachment points 44 of the support element 34 (see Fig. 4).

In the example depicted in Figs. 2 and 3, the number of the suspension rods 12 corresponds to the number of connecting elements 3. Likewise, the number of fixed sections may correspond to the number of displaceable sections. However, it is conceivable that the number of suspension rods 12 is dissimilar from the number of connecting elements 43. Furthermore, the number of fixed sections may differ from the number of displaceable sections.

Fig. 4 illustrates an embodiment of an inventive magnet support structure 30 for use in a "wet" magnetic resonance device. In the depicted example, the magnet support structure 30 comprises an outer vacuum chamber 42, a support element 34 and a cryogen vessel 31 containing the main magnet 17. The support element 34 is suspended of the outer vacuum chamber 42 via suspension rods 12. The cryogen vessel 31 is mechanically connected to the support element 34 via three connecting elements 43. The main magnet 17 is mechanically supported within the cryogen vessel 31 (not shown).

Preferably, the magnet support structure 30 depicted in Fig. 4 is used in a "wet" magnetic resonance device. However, instead of a cryogen vessel 31, the support element 34 may be directly connected to a main magnet 17. Such a configuration of the magnet support system 30 may preferably be used in "dry" magnetic resonance devices.

In the embodiment depicted in Fig. 4, a plurality of attachment points 44 protrude from a lateral surface of the support element 34. The attachment points 44a, 44b and the attachment points 44c, 44d are arranged at opposite sides of the support element 34. The suspension rods 12 provide a mechanical connection between the outer shell of the vacuum chamber 42 and the attachment points 44. The suspension rods 12 are oriented at a smallest angle *α* with respect to the gravitational force vector 81. In the depicted embodiment, the angle *α* is at least 35°.

The attachment points 44 may be formed as anchors. The end sections of the suspension rods 12 may comprise ferrules (not shown) configured to mechanically engage with the attachment points 44. For example, the end sections of the suspension rods 12 may comprise a male thread configured to engage with a female thread of the attachment points 44. However, any suitable mechanical connection may be used to mechanically connect the suspension rods 12 to the attachment points 44.

The support element 34 comprises three fixed sections 34a and three displaceable sections 34b. The displaceable sections 34b are configured to be bend or displaced when a force, particularly a suspension load, is applied to at least one attachment point 44. The cold mass, i. e. the main magnet 17 or the cryogen vessel 31, is spaced from the support element 34 via the connecting elements 43 in such a way, that a mechanical contact between the displaceable sections 34b and the cold mass 17, 31 is avoided when the displaceable sections 34b are displaced or deflected. Preferably, the displaceable sections 34b are deflected or displaced in a radial direction of the support element 34.

The support element 34 is configured in such a way, that a predefined relative spatial arrangement of the fixed sections 34a is maintained when the force is applied to the attachment points 44. Thus, a warping or deforming of the cold mass 17, 31 mechanically connected to the support element 34 via the connecting elements 43 can favourably be prevented.

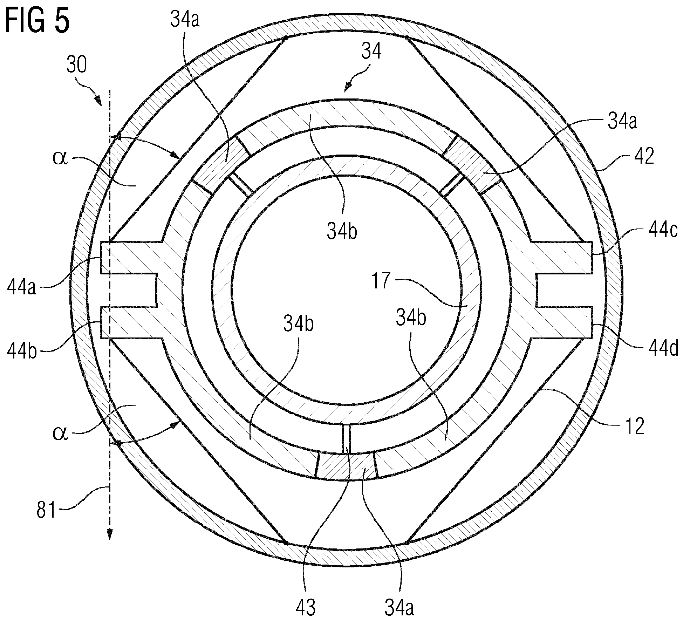

Fig. 5 shows a further embodiment of the inventive magnet support structure 30. In the depicted example, the smallest angle *α* between the suspension rods 12 and the gravitational force vector 81 is increased in comparison to the embodiment illustrated in Fig. 4. For example, the smallest angle *α* may be at least 40° or 45°. The shallower angle *α* of the embodiment shown in Fig. 5 may favourably decrease a tendency of the cold mass 17, 31 to roll and/or twist in relation to the support element 34 in comparison to the embodiment depicted in Fig. 4. A rolling or twisting motion of the cold mass 17, 31 may be caused by a force applied to the attachment points 44 or by electromagnetic forces associated with a magnetic resonance measurement via the magnetic resonance device 11.

Although the fixed sections 34a in Figs. 4 and 5 are highlighted via a hatched area, the displaceable sections 34b, the fixed sections 34a and the attachment points 44 may form a monolithic structure. It is also conceivable, however, that the attachment points 44 are mounted to the support structure 34. In a less preferable embodiment of the support structure 34, the fixed sections 34a and the displaceable sections 34b are separate components mechanically connected to form the support element 34.

Fig. 6 illustrates an embodiment of the support element 34. In the depicted example, the displaceable sections 34b, the fixed sections 34a and the attachment points 44 form a monolithic structure. The support element 34 comprises the shape of a ring or a tube.

The support element 34 shown in Fig. 6 has been examined in a mechanical strain simulation or load simulation. A force was applied to the attachment points 44a, 44b, 44c and 44d (44a-d) and the resulting displacement of sections of the support structure 34 has been determined and is visualized via the colour axis C. For the simulation, a shock was assumed which corresponded to a force of 5-times the force of gravity. The shock was assumed to accelerate the support element 34 in the Y-direction, to simulate a drop of the support element 34.

Fig. 6 shows an exemplary visualization of a deformation pattern. The attachment points 44b and 44d absorb most of the shock and are displaced by a maximum of about 3 mm. The displaceable sections 34b are affected to a lesser extent, showing a maximum displacement of about 2.2 mm. However, the fixed sections 34a remain in their predefined relative spatial arrangement and show no displacement at all. Thus, a cold mass mechanically connected to the connecting elements 43 arranged at the fixed sections 34a would not be affected by the shock.

The deformation pattern or the information contained in the deformation pattern depicted in Fig. 6 may be used in an inventive computer-implemented method for designing a support element (34) according to an embodiment described above.

The embodiments described herein are to be recognized as examples. It is to be understood that individual embodiments may be extended by or combined with features of other embodiments if not stated otherwise. The embodiments depicted in the Figs. 1 to 9 are representations that do not necessarily have to be to scale.

## Claims

1. Support element (34) for supporting a cold mass (17; 31) of a magnetic resonance device (11), comprising at least one attachment point (44) which provides a mounting element configured for mechanically connecting the support element (34) to a support structure, a plurality of displaceable sections (34b) and a plurality of fixed sections (34a), wherein each displaceable section of the plurality of displaceable sections (34b) is configured to be reversibly displaced relative to fixed sections of the plurality of fixed sections (34a) when a predefined force is applied to the at least one attachment point (44) and wherein the fixed sections of the plurality fixed sections (34a) are configured to remain in a predefined relative spatial arrangement to each other when the predefined force is applied to the at least one attachment point (44), wherein the predefined force is oriented in at least one spatial direction, **characterized in that** the support element (34) comprises the shape of a ring, a hollow cylinder, a hollow prism, a tube, or a disc.

2. Support element (34) according to claim 1, wherein the at least one attachment point (44) is arranged at a displaceable section of the plurality of displaceable sections (34b) and mechanically connected to the displaceable section.

3. Support element (34) according to one of the preceding claims, comprising at least one connecting element (43) configured to mechanically connect to an inert mass, wherein the at least one connecting element (43) is arranged at a fixed section of the plurality of fixed sections (34a) and mechanically connected to the fixed section.

4. Support element (34) according to one of the preceding claims, wherein the plurality of displaceable sections (34b) comprises a first displaceable section and a second displaceable section and wherein the first displaceable section and the second displaceable section are separated by a fixed section of the plurality of fixed sections (34a).

5. Support element (34) according to one of the preceding claims, wherein the support element (34) comprises at least three displaceable sections (34b) and at least three fixed sections (34a).

6. Support element (34) according to a preceding claim, wherein the support element (34) comprises the shape of a tube, a ring, or a disc and wherein the at least one attachment point (44) protrudes from a lateral surface of the support element (34).

7. Support element (34) according to claim 6, comprising at least two attachment points (44) arranged at opposing sides of the support element (34).

8. Support element (34) according to a preceding claim, wherein a vector of the predefined force is oriented in parallel to a vector of the gravitational force, wherein the support element (34) comprises the shape of a tube or a ring and wherein the plurality of displaceable sections (34b) is configured to be displaced along a radial direction of the support element (34) when the predefined force is applied to the at least one attachment point (44).

9. Magnet support structure (30), comprising an outer vacuum chamber (42), a cold mass (17; 31), and a support element (34) according to one of the preceding claims, wherein the at least one attachment point (44) is mechanically connected to the outer vacuum chamber (42) and the plurality of fixed sections (34a) is mechanically connected to the cold mass (17; 31), and wherein the cold mass (17; 31) is spaced from the plurality of displaceable sections (34b).

10. Magnet support structure (30) according to claim 9, wherein the at least one attachment point (44) is mechanically connected to the outer vacuum chamber (42) via at least one suspension element (12).

11. Magnet support structure (30) according to one of the claims 9 or 10, wherein the plurality of fixed sections (34a) is mechanically connected to the cold mass (17; 31) via a plurality of connecting elements (43) and wherein the plurality of connecting elements (43) is configured to space the support element (34) from the cold mass (17; 31).

12. Magnetic resonance device (11) for acquiring magnetic resonance data of an object positioned within an imaging region (36) of the magnetic resonance device (11), comprising a magnet support structure (30) according to one of the claims 9 to 11.

13. Magnetic resonance device (11) according to claim 12 comprising a magnet support structure (30) according to claim 10, wherein a smallest angle between a line defined by an axial extension of the at least one suspension element (12) and a gravitational force vector (81) is at least 35°.

14. Computer-implemented method for designing a support element (34) according to one of the claims 1 to 8, comprising the steps:
• performing a load simulation of the support element (34) to determine a deformation pattern of the support element (34) when a predefined force is applied to at least one attachment point (44), wherein an inert mass is mechanically connected to the support element (34) at a plurality of sections arranged at predefined spatial locations, and
• determining a parameter of the support element (34) in dependence of the determined deformation pattern to provide a support element (34) comprising a plurality of displaceable sections (34b) and a plurality of fixed sections (34a) arranged at the predefined spatial locations,
wherein each displaceable section of the plurality of displaceable sections (34b) is configured to be reversibly displaced relative to fixed sections of the plurality of fixed sections (34a) when a predefined force is applied to the at least one attachment point (44) and wherein the fixed sections of the plurality fixed sections (34a) are configured to remain in a predefined relative spatial arrangement to each other when the predefined force is applied to the at least one attachment point (44).

## Patentansprüche

1. Trägerelement (34) zum Tragen einer kalten Masse (17; 31) einer Magnetresonanzvorrichtung (11), umfassend mindestens einen Befestigungspunkt (44), der ein Montageelement bereitstellt, das zum mechanischen Verbinden des Trägerelements (34) mit einer Trägerstruktur ausgelegt ist, eine Vielzahl von verschiebbaren Abschnitten (34b) und eine Vielzahl von festen Abschnitten (34a), wobei jeder verschiebbare Abschnitt der Vielzahl von verschiebbaren Abschnitten (34b) dazu ausgelegt ist, bezogen auf die festen Abschnitte der Vielzahl von festen Abschnitten (34a) reversibel verschoben zu werden, wenn eine vordefinierte Kraft auf den mindestens einen Befestigungspunkt (44) aufgebracht wird, und wobei die festen Abschnitte der Vielzahl von festen Abschnitten (34a) dazu ausgelegt sind, in einer vordefinierten relativen räumlichen Anordnung zueinander zu verbleiben, wenn die vordefinierte Kraft auf den mindestens einen Befestigungspunkt (44) aufgebracht wird, wobei die vordefinierte Kraft in mindestens einer räumlichen Richtung ausgerichtet ist, **dadurch gekennzeichnet, dass** das Trägerelement (34) die Form eines Rings, eines Hohlzylinders, eines Hohlprismas, einer Röhre oder einer Scheibe umfasst.

2. Trägerelement (34) nach Anspruch 1, wobei der mindestens eine Befestigungspunkt (44) an einem verschiebbaren Abschnitt der Vielzahl von verschiebbaren Abschnitten (34b) angeordnet und mechanisch mit dem verschiebbaren Abschnitt verbunden ist.

3. Trägerelement (34) nach einem der vorhergehenden Ansprüche, umfassend mindestens ein Verbindungselement (43), das dazu ausgelegt ist, sich mechanisch mit einer inerten Masse zu verbinden, wobei das mindestens eine Verbindungselement (43) an einem festen Abschnitt der Vielzahl von festen Abschnitten (34a) angeordnet und mechanisch mit dem festen Abschnitt verbunden ist.

4. Trägerelement (34) nach einem der vorhergehenden Ansprüche, wobei die Vielzahl von verschiebbaren Abschnitten (34b) einen ersten verschiebbaren Abschnitt und einen zweiten verschiebbaren Abschnitt umfasst und wobei der erste verschiebbare Abschnitt und der zweite verschiebbare Abschnitt durch einen festen Abschnitt der Vielzahl von festen Abschnitten (34a) getrennt sind.

5. Trägerelement (34) nach einem der vorhergehenden Ansprüche, wobei das Trägerelement (34) mindestens drei verschiebbare Abschnitte (34b) und mindestens drei feste Abschnitte (34a) umfasst.

6. Trägerelement (34) nach einem der vorhergehenden Ansprüche, wobei das Trägerelement (34) die Form einer Röhre, eines Rings oder einer Scheibe umfasst und wobei der mindestens eine Befestigungspunkt (44) von einer Seitenfläche des Trägerelements (34) vorsteht.

7. Trägerelement (34) nach Anspruch 6, umfassend mindestens zwei Befestigungspunkte (44), die auf gegenüberliegenden Seiten des Trägerelements (34) angeordnet sind.

8. Trägerelement (34) nach einem vorhergehenden Anspruch, wobei ein Vektor der vordefinierten Kraft parallel zu einem Vektor der Schwerkraft ausgerichtet ist, wobei das Trägerelement (34) die Form einer Röhre oder eines Rings umfasst und wobei die Vielzahl von verschiebbaren Abschnitten (34b) dazu ausgelegt ist, entlang einer radialen Richtung des Trägerelements (34) verschoben zu werden, wenn die vordefinierte Kraft auf den mindestens einen Befestigungspunkt (44) aufgebracht wird.

9. Magnetträgerstruktur (30), umfassend eine äußere Vakuumkammer (42), eine kalte Masse (17; 31) und ein Trägerelement (34) nach einem der vorhergehenden Ansprüche, wobei der mindestens eine Befestigungspunkt (44) mechanisch mit der äußeren Vakuumkammer (42) verbunden und die Vielzahl von festen Abschnitten (34a) mechanisch mit der kalten Masse (17; 31) verbunden ist, und wobei die kalte Masse (17; 31) von der Vielzahl von verschiebbaren Abschnitten (34b) beabstandet ist.

10. Magnetträgerstruktur (30) nach Anspruch 9, wobei der mindestens eine Befestigungspunkt (44) über mindestens ein Aufhängungselement (12) mechanisch mit der äußeren Vakuumkammer (42) verbunden ist.

11. Magnetträgerstruktur (30) nach einem der Ansprüche 9 oder 10, wobei die Vielzahl von festen Abschnitten (34a) über eine Vielzahl von Verbindungselementen (43) mechanisch mit der kalten Masse (17; 31) verbunden ist und wobei die Vielzahl von Verbindungselementen (43) dazu ausgelegt ist, das Trägerelement (34) von der kalten Masse (17; 31) zu beabstanden.

12. Magnetresonanzvorrichtung (11) zum Erfassen von Magnetresonanzdaten eines in einer Bildgebungsregion (36) der Magnetresonanzvorrichtung (11) positionierten Objekts, umfassend eine Magnetträgerstruktur (30) nach einem der Ansprüche 9 bis 11.

13. Magnetresonanzvorrichtung (11) nach Anspruch 12, umfassend eine Magnetträgerstruktur (30) nach Anspruch 10, wobei ein kleinster Winkel zwischen einer Linie, die von einer axialen Erstreckung des mindestens einen Aufhängungselements (12) definiert wird, und einem Schwerkraftvektor (81) mindestens 35° beträgt.

14. Computerimplementiertes Verfahren zum Entwerfen eines Trägerelements (34) nach einem der Ansprüche 1 bis 8, umfassend die Schritte:
• Durchführen einer Belastungssimulation des Trägerelements (34), um ein Verformungsmuster des Trägerelements (34) zu bestimmen, wenn eine vordefinierte Kraft auf mindestens einen Befestigungspunkt (44) aufgebracht wird, wobei eine inerte Masse an einer Vielzahl von Abschnitten, die an vordefinierten räumlichen Orten angeordnet sind, mechanisch mit dem Trägerelement (34) verbunden ist, und
• Bestimmen eines Parameters des Trägerelements (34) in Abhängigkeit von dem bestimmten Verformungsmuster, um ein Trägerelement (34) bereitzustellen, das eine Vielzahl von verschiebbaren Abschnitten (34b) und eine Vielzahl von festen Abschnitten (34a) umfasst, die an den vordefinierten räumlichen Orten angeordnet sind,
wobei jeder verschiebbare Abschnitt der Vielzahl von verschiebbaren Abschnitten (34b) dazu ausgelegt ist, bezogen auf feste Abschnitte der Vielzahl von festen Abschnitten (34a) reversibel verschoben zu werden, wenn eine vordefinierte Kraft auf den mindestens einen Befestigungspunkt (44) aufgebracht wird, und wobei die festen Abschnitte der Vielzahl von festen Abschnitten (34a) dazu ausgelegt sind, in einer vordefinierten relativen räumlichen Anordnung zueinander zu verbleiben, wenn die vordefinierte Kraft auf den mindestens einen Befestigungspunkt (44) aufgebracht wird.

## Revendications

1. Elément (34) de support pour supporter une masse (17 ; 31) froide d'un dispositif (11) de résonnance magnétique, comprenant au moins un point (44) de fixation, qui donne un élément de montage configuré pour assembler mécaniquement l'élément (34) de support à une structure de support, une pluralité de parties (34b) déplaçables et une pluralité de parties (34a) fixes, dans lequel chaque partie déplaçable de la pluralité de parties (34b) déplaçables est configurée pour être déplacée réversiblement par rapport à des parties fixes de la pluralité de parties (34a) fixes, lorsqu'une force définie à l'avance est appliquée au au moins un point (44) de fixation et dans lequel les parties fixes de la pluralité des parties (34a) fixes sont configurées pour rester dans une disposition spatiale relative définie à l'avance les unes par rapport aux autres, lorsque la force définie à l'avance est appliquée au au moins un point (44) de fixation, dans lequel la force définie à l'avance est orientée dans au moins une direction de l'espace, **caractérisé en ce que** l'élément de support (34) de support comprend la forme d'un anneau, d'un cylindre creux, d'un prisme creux, d'un tube ou d'un disque.

2. Elément (34) de support suivant la revendication 1, dans lequel le au moins un point (44) de fixation est disposé sur une partie déplaçable de la pluralité de parties (34b) déplaçables et est assemblé mécaniquement à la partie déplaçable.

3. Elément (34) de support suivant l'une des revendications précédentes, comprenant au moins un élément (43) d'assemblage configuré pour s'assembler mécaniquement à une masse inerte, dans lequel le au moins un élément (43) d'assemblage est disposé à une partie fixe de la pluralité de parties (34a) fixes et est assemblé mécaniquement à la partie fixe.

4. Elément (34) de support suivant l'une des revendications précédentes, dans lequel la pluralité de parties (34b) déplaçables comprend une première partie déplaçable et une deuxième partie déplaçable et dans lequel la première partie déplaçable et la deuxième partie déplaçable sont séparées par une partie fixe de la pluralité de parties (34a) fixes.

5. Elément (34) de support suivant l'une des revendications précédentes, dans lequel l'élément (34) de support comprend au moins trois parties (34b) déplaçables et au moins trois parties (34a) fixes.

6. Elément (34) de support suivant une revendication précédente, dans lequel l'élément (34) de support comprend la forme d'un tube, d'un anneau ou d'un disque et dans lequel le au moins un point (44) de fixation fait saillie d'une surface latérale de l'élément (34) de support.

7. Elément (34) de support suivant la revendication 6, comprenant au moins deux points (44) de fixation disposés sur des côtés opposés de l'élément (34) de support.

8. Elément (34) de support suivant une revendication précédente, dans lequel un vecteur de la force définie à l'avance est orienté parallèlement à un vecteur de la force de gravité, dans lequel l'élément (34) de support comprend la forme d'un tube ou d'un anneau et dans lequel la pluralité de parties (34b) déplaçables est configurée pour être déplacée suivant une direction radiale de l'élément (34) de support, lorsque la force définie à l'avance est appliquée au au moins un point (44) de fixation.

9. Structure (30) de support d'aimant, comprenant une chambre (42) extérieure sous vide, une masse (17 ; 31) froide et un élément (34) de support suivant l'une des revendications précédentes, dans lequel le au moins un point (44) de fixation est assemblé mécaniquement à la chambre (42) extérieure sous vide et la pluralité de parties (34a) fixées est assemblé mécaniquement à la masse (17 ; 31) froide, dans lequel la masse (17 ; 31) froide est à distance de la pluralité de parties (34b) déplaçables.

10. Structure (30) de support d'aimant suivant la revendication 9, dans laquelle le au moins un point (44) de fixation est assemblé mécaniquement à la chambre (42) extérieure sous vide par l'intermédiaire d'au moins un élément (12) de suspension.

11. Structure (30) de support d'aimant suivant l'une des revendications 9 ou 10, dans lequel la pluralité de parties (34a) fixes est assemblée mécaniquement à la masse (17 ; 31) froide par l'intermédiaire d'une pluralité d'éléments (43) de liaison et dans laquelle la pluralité d'éléments (43) de liaison est configurée pour mettre l'élément (34) de support à distance de la masse (17 ; 31) froide.

12. Dispositif (11) de résonnance magnétique pour acquérir des données de résonnance magnétique d'un objet placé dans une région (36) d'imagerie du dispositif (11) de résonnance magnétique, comprenant la structure (30) de support d'aimant suivant l'une quelconque des revendications 9 à 11.

13. Dispositif (11) de résonnance magnétique suivant la revendication 12, comprenant une structure (30) de support d'aimant suivant la revendication 10, dans lequel un angle le plus petit, entre une ligne, définie par une étendue axiale du au moins un élément (12) de suspension et un vecteur (81) de la force de gravité, est d'au moins 35°.

14. Procédé mis en œuvre par ordinateur pour concevoir un élément (34) de support suivant l'une des revendications 1 à 8, comprenant les stades :
• effectuer une simulation de charge de l'élément (34) de support pour déterminer une configuration de déformation de l'élément (34) de support, lorsque une force définie à l'avance est appliquée à au moins un point (44) de fixation, dans lequel une masse inerte est assemblée mécaniquement à l'élément (34) de support à une pluralité de parties disposées en des emplacements définis à l'avance dans l'espace, et
• déterminer un paramètre de l'élément (34) de support en fonction de la configuration de déformation déterminée pour donner un élément (34) de support comprenant une pluralité de parties (34b) déplaçables et une pluralité de parties (34a) fixes disposées à des emplacements définis à l'avance dans l'espace,
dans lequel chaque partie déplaçable de la pluralité de parties (34b) déplaçables est configurée pour être déplacée réversiblement par rapport à des parties fixes de la pluralité de parties (34a) fixes, lorsqu'une force définie à l'avance est appliquée au au moins un point (44) de fixation et dans lequel les parties fixes de la pluralité de parties (34a) fixes sont configurées pour rester dans une disposition relative dans l'espace définie à l'avance les unes par rapport aux autres, lorsque la force définie à l'avance est appliquée au au moins un point (44) de fixation.
